# FASCICULE DE BREVET EUROPEEN

(11) **EP 2 839 813 B1**
(45) Date de publication et mention de la délivrance du brevet: **15.06.2016**
(21) Numéro de dépôt: 13354029.4
(22) Date de dépôt: 22.08.2013
(51) Int. Cl.: A61F 2/80, A61F 2/50

(54) **Bouchon à valve automatique pour une emboîture de prothèse à dépression**
Kappe mit einem automatischen Ventil für einen unterdruckunterstützten Prothesenschaft
Plug with automatic valve for a prosthetic vacuum-assisted socket

(43) Date de publication de la demande: 25.02.2015
(73) Titulaire: Chabloz Composants, 38170 Seyssinet-Pariset (FR)
(72) Inventeur: Chabloz, Pierre, 38450 Saint-Georges-de-Commiers (FR)
(74) Mandataire: Hecké, Gérard

(56) Documents cités:
- EP-A1- 1 875 881
- DE-C- 605 534
- US-A- 2 569 790

## Description

### Domaine technique de l'invention

L'invention est relative à un bouchon à valve automatique pour une emboîture de prothèse à dépression, ledit bouchon comportant :
- une embase de fixation munie d'un embout tubulaire destiné à être assemblé à un orifice de l'emboîture en forme de coque,
- un élément support de la valve d'évacuation d'air hors de la coque, et d'interdiction d'entrée d'air à l'intérieur de la coque, ledit élément support étant articulé à l'embase, et étant mobile par pivotement entre une position rapprochée où l'élément support est plaqué contre l'embase, et une position écartée où la valve est éloignée de l'orifice de manière à faire communiquer l'intérieur de la coque avec l'extérieur,
- ladite valve ayant un organe souple évidé en biseau dont la fente est normalement fermée, et qui s'ouvre, sous l'effet de l'air chassé hors de la coque.

Un tel bouchon est connu, par exemple, du document EP 1875881.

### État de la technique

Dans les prothèses à maintien par dépression, il est connu d'organiser la coque de manière à permettre une mise en place et un retrait du moignon qui soient simples et rapides, tout en garantissant une fiabilité de la jonction par emboîtement obtenue entre le moignon et la prothèse. A cet effet, il a été proposé d'organiser un emboîtement étanche à l'air entre le moignon et la coque pour interdire leur désolidarisation spontanée. Le moignon est préalablement enveloppé d'un manchon de forme extérieure complémentaire à la cavité de la coque, pour interdire un passage d'air entre eux et en conséquence retenir spontanément le moignon à l'intérieur de la coque. Pour autoriser la mise en place du moignon à l'intérieur de la coque, l'air résiduel contenu dans la cavité est chassé à travers un évent ménagé à la base de la coque. Cet évent est équipé d'un bouchon amovible qui est prévu pour être mis en place après l'introduction du moignon à l'intérieur de la coque en vue d'interdire une introduction d'air à l'intérieur de la cavité, et qui est prévu pour être retiré en vue d'autoriser une entrée d'air à l'intérieur de la cavité lorsque l'utilisateur souhaite enlever la prothèse. Pour garantir l'étanchéité à l'air entre le manchon et la coque, un organe d'étanchéité est placé à l'extrémité proximale de la coque. Par exemple, cet organe d'étanchéité est constitué d'un joint à lèvre qui est interposé entre la coque et le manchon en étant indifféremment fixé sur l'un de ceux-ci.

La mise en place et le retrait de la prothèse restent néanmoins contraignantes pour l'utilisateur. Plus particulièrement, les manipulations répétées du bouchon sont incommodes et inconfortables.

Le document EP 1875881 propose d'articuler le bouchon sur la coque à laquelle il reste solidarisé entre les positions d'obturation et de libération de l'évent. Un organe de rappel du type ressort de torsion, sollicite le bouchon en position d'obturation de l'évent. Pour la mis en place de la prothèse, l'utilisateur devra néanmoins maintenir le clapet ouvert en appuyant sur le prolongement de la branche mobile. Cette action manuelle d'ouverture forcée du clapet doit se poursuivre jusqu'à l'introduction complète du moignon et du manchon dans la coque. Une telle manipulation peut s'avérer peu pratique pour certains utilisateurs.

### Objet de l'invention

L'objet de l'invention consiste à réaliser un bouchon à valve automatique pour une emboîture de prothèse à dépression, dont la mise en oeuvre est rapide et facile pour l'utilisateur, et dont la fabrication est peu coûteuse.

Le bouchon à valve selon l'invention, défini dans la revendication 1, est caractérisé en ce que l'embase du bouchon est équipée de plus :
- d'un organe d'encliquetage coopérant avec un bec de retenu de l'élément support de manière à assurer un verrouillage positif du bouchon en position rapprochée pour relier la valve à l'orifice,
- et d'une patte d'actionnement pour assurer le déverrouillage du bec de retenue de l'élément support lors du déplacement du bouchon vers la position écartée.

Lorsque l'utilisateur appuie sur l'élément support, la valve se trouve en aboutement avec l'orifice de la coque, et l'extraction d'air s'effectue à faible débit à travers la valve. Le claquement du verrouillage permet à l'utilisateur de savoir s'il a bien fermé le bouchon.

Pour ouvrir la valve, un simple ordre de déclenchement sur la patte d'actionnement est suffisant pour assurer le déverrouillage de l'élément support, lequel est sollicité automatiquement vers la position écartée de libération de l'orifice.

Dans les deux utilisations, le bouchon avec la valve reste toujours solidarisé à la coque, et est de fait imperdable.

Selon un mode de réalisation préférentiel, l'élément support de la valve est sollicité élastiquement vers la position écartée lors dudit déverrouillage. A cet effet, un ressort, notamment un ressort de torsion, peut être logé dans la zone d'articulation de l'élément support et de l'embase. L'effet ressort peut également résulter de la conformation du bouchon réalisé par une pièce plastique injectée.

De préférence, la valve est montée sur la partie supérieure de l'élément support, et est surmontée d'un capot percé par au moins un trou. La présence de ce capot facilite le déplacement de la valve vers la position rapprochée.

Selon une autre caractéristique, l'organe d'encliquetage et le bec de retenue se trouvent à l'opposé de la zone d'articulation de l'élément support.

### Description sommaire des dessins

D'autres avantages et caractéristiques ressortiront plus clairement de la description qui va suivre d'un mode de réalisation de l'invention donné à titre d'exemple non limitatif et représenté aux dessins annexés, dans lesquels :
- la figure 1 est une vue schématique en coupe de la coque équipée du bouchon selon l'invention, le manchon étant représenté en position inséré dans la coque, et le bouchon étant fermé avec aboutement de la valve contre l'orifice de la coque ;
- la figure 2 est une vue identique de la figure 1, après retrait du manchon, et ouverture du bouchon ;
- les figures 3 et 5 sont des vues en coupe du bouchon, respectivement en position rapprochée verrouillée, et en position écartée déverrouillée ;
- la figure 4 représente une vue en plan de la figure 3.

### Description détaillée de l'invention

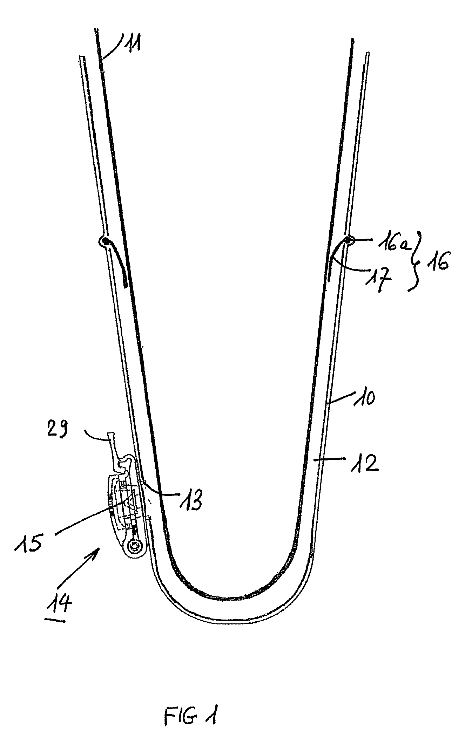

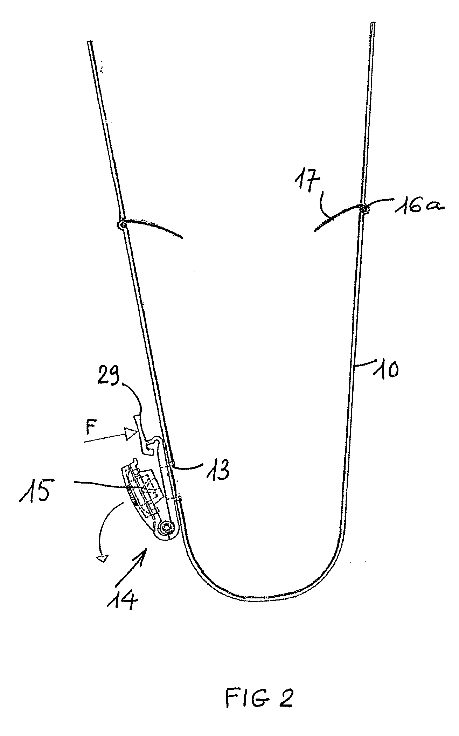

Sur les figures 1 et 2, une coque 10 de prothèse est destinée à recevoir par emboîtement étanche un manchon 11 installé préalablement sur le moignon d'un membre amputé, par exemple un bras ou une jambe. La coque 10 présente une forme en U à fond fermé, et à entrée évasée ouverte, de manière à délimiter une cavité 12 semi-ouverte de réception du manchon 11.

La coque 10 est dotée à sa base d'un orifice 13 ou évent dans lequel est introduit un bouchon 14 équipé d'une valve 15 à expulsion d'air. Le bouchon 14 est manoeuvrable par pivotement entre une position fermée d'obturation et une position ouverte de libération de l'orifice 13. En position d'obturation du bouchon 14, la valve 15 qui est intégrée dans le bouchon 14, permet d'évacuer l'air hors de la cavité 12, en interdisant toute entrée d'air à l'intérieur de la cavité 12.

Des moyens à dépression sont utilisés pour retenir la coque 10 sur le manchon 11. Un joint 16 d'étanchéité interposé entre la coque 10 et le manchon 11, est formé par un anneau 16a à lèvre 17 souple, logé dans une rainure annulaire de la coque 10. Un exemple de réalisation d'un tel joint 16 est décrit dans le document EP 1875882. La présence de ce joint 16 s'oppose à tout passage d'air dans la cavité 12 lors de l'introduction du manchon 11.

En position ouverte du bouchon 14 (figure 2), l'air présent dans la cavité 12 est expulsé avec un gros débit à travers l'orifice 13 pour que la mise en place ou le retrait de la prothèse puissent s'effectuer rapidement et avec facilité.

En position fermée du bouchon 14 (figure 1) après mise en place et utilisation de la prothèse, la valve 15 expulse l'air vers l'extérieur avec un faible débit, suite à une poussée d'air en provenance de la cavité 12.

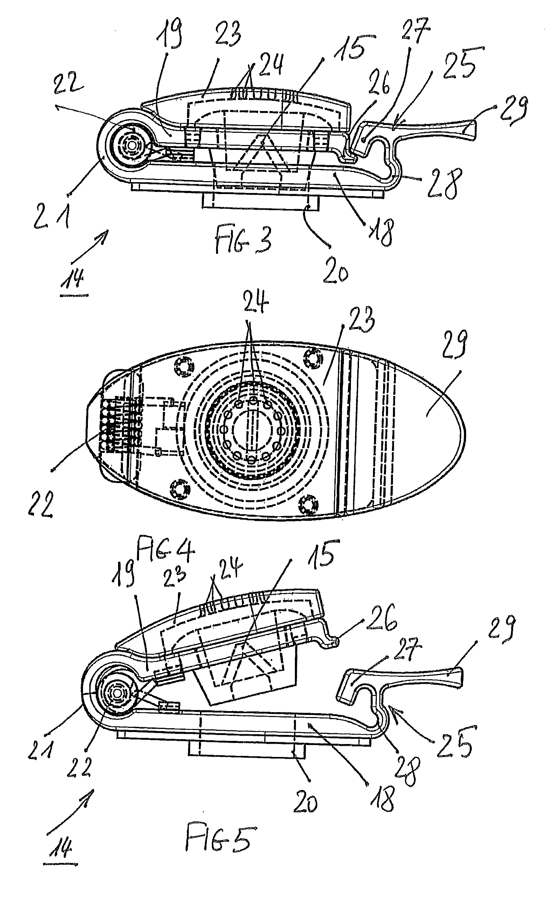

En référence aux figures 3 à 5, le bouchon 14 est formé par une pièce en matière plastique injectée, comportant une embase 18 de fixation à la coque 10, et d'un élément support 19 de la valve 15. L'embase 18 est munie d'un embout 20 tubulaire destiné à être assemblé par emboîtement dans l'orifice 13 en solidarisant le bouchon à la coque 13. L'élément support 19 de la valve 15 est monté articulé, par exemple au moyen d'une charnière 21, sur l'embase 18 entre une position rapprochée (figure 3) et une position écartée (figure 5). Dans la position rapprochée, l'élément support 19 est plaqué contre l'embase 18 en faisant communiquer directement la valve 15 avec l'embout 20 et l'orifice 13 d'accès à la cavité 12. Dans la position écartée, la valve 15 est éloignée de l'embout 20, de manière à relier l'intérieur de la cavité 12 avec l'extérieur.

Un ressort 22 est logé dans la zone d'articulation de la charnière 21, et sollicite élastiquement l'élément support 19 vers la position écartée. Le ressort 22 représenté aux figures 3 à 5, est à titre d'exemple, un ressort de torsion, mais tout autre type d'organe élastique de rappel peut être utilisé dans la zone d'articulation. L'effet ressort peut également résulter de la conformation de la pièce plastique injectée.

La valve 15 est du type anti-retour, et comporte un organe souple évidé conformé en biseau orienté vers l'extérieur. Elle est munie d'une fente en bout du biseau pour le passage de l'air en provenance de la cavité 12. La fente de la valve 15 est normalement fermée, et s'ouvre par déformation du biseau sous l'effet d'une augmentation de pression suite à la poussée d'air chassé hors de la cavité 12. En l'absence d'une telle pression d'air, la valve 15 reste fermée, et interdit toute introduction d'air à travers l'orifice 13.

La valve 15 est clipsée sur la partie supérieure de l'élément support 19, et est surmontée d'un capot 23 percé par une pluralité de trous 24 en liaison avec la valve 15.

L'embase 18 du bouchon 14 est équipée en plus d'un organe d'encliquetage 25 coopérant en position verrouillée avec un bec de retenue 26 de l'élément support 19. L'organe d'encliquetage 25 se trouve à l'opposé de la charnière 21, et est conformé en crochet 27 relié à l'extrémité de l'embase 18 par une liaison souple 28. Une patte d'actionnement 29 est fixée au crochet 27 pour assurer le déverrouillage du bec de retenue 26 lors du déplacement du bouchon 14 vers la position écartée de la figure 2.

Après mise en place de l'embout 20 dans l'orifice 13 de la coque 10, le fonctionnement du bouchon 14 est le suivant :
Une poussée sur le capot 23 provoque le déplacement de l'élément support 19 contre l'embase 18 à l'encontre de la force de rappel du ressort 22. La valve 15 se trouve en regard de l'orifice 13, et permet d'extraire l'air de la cavité 12 avec un petit débit. L'entrée d'air vers la cavité 12 n'est pas possible.

Pour ouvrir le bouchon 14, il suffit d'appuyer sur la patte d'actionnement 29 dans le sens de la flèche F (figure 2) pour libérer le bec de retenue 26 de l'action de verrouillage du crochet 27 de l'organe d'encliquetage. Cette action de déverrouillage est possible grâce à la déformation élastique de la liaison souple 28. L'élément support 19 avec la valve 15 est sollicité par la détente du ressort 22 vers la position écartée, de manière à libérer l'orifice 13 de la coque. L'air est expulsé avec un gros débit, et permet la mise en place ou le retrait de la prothèse.

## Revendications

1. Bouchon (14) à valve (15) automatique pour une emboîture de prothèse à dépression en forme de coque (10), ledit bouchon (14) comportant :
- une embase (18) de fixation munie d'un embout (20) tubulaire destiné à être assemblé à un orifice (13) de l'emboîture en forme de coque (10),
- un élément support (19) de la valve (15) d'évacuation d'air hors de la coque, et d'interdiction d'entrée d'air à l'intérieur de la coque (10), ledit élément support (19) étant articulé à l'embase (18), et étant mobile par pivotement entre une position rapprochée où l'élément support (19) est plaqué contre l'embase (18), et une position écartée où la valve (15) est éloignée de l'orifice (13) de manière à faire communiquer l'intérieur de la coque (10) avec l'extérieur,
- ladite valve (15) ayant un organe souple évidé en biseau dont la fente est normalement fermée, et qui s'ouvre, sous l'effet de l'air chassé hors de la coque,
**caractérisé en ce que** l'embase (18) du bouchon (14) est équipée de plus :
- d'un organe d'encliquetage (25) coopérant avec un bec de retenue (26) de l'élément support (19) de manière à assurer un verrouillage positif du bouchon (14) en position rapprochée pour relier la valve (15) à l'orifice (13),
- et d'une patte d'actionnement (29) pour assurer le déverrouillage du bec de retenue (26) de l'élément support (19) lors du déplacement du bouchon (14) vers la position écartée.

2. Bouchon à valve selon la revendication 1, **caractérisé en ce que** l'élément support (19) de la valve (15) est sollicité élastiquement vers la position écartée lors dudit déverrouillage.

3. Bouchon à valve selon la revendication 2, **caractérisé en ce qu'**un ressort (22) est logé dans la zone d'articulation de l'élément support (19) et de l'embase (18).

4. Bouchon à valve selon la revendication 3, **caractérisé en ce que** le ressort (22) est un ressort de torsion.

5. Bouchon à valve selon l'une des revendications précédentes, **caractérisé en ce que** la valve (15) est montée sur la partie supérieure de l'élément support (19), et est surmontée d'un capot (23) percé par au moins un trou (24).

6. Bouchon à valve selon l'une des revendications précédentes, **caractérisé en ce que** l'organe d'encliquetage (25) et le bec de retenue (26) se trouvent à l'opposé de la zone d'articulation de l'élément support (19).

7. Bouchon à valve selon l'une des revendications précédentes, **caractérisé en ce que** l'élément support (19) et l'embase (18) sont formés par une seule pièce en matière plastique injectée.

8. Coque (10) d'une prothèse destinée à recevoir un manchon (11) installé sur le moignon d'un membre amputé, **caractérisée en ce qu'**elle est équipée d'un bouchon (14) à valve (15) selon l'une des revendications précédentes.

## Patentansprüche

1. Verschluss (14) mit einem automatischen Ventil (15) für ein schalenförmiges (10) Unterdruck-Prothesen-Steck-Element, welcher Verschluss (14) umfasst:
- einen Befestigungssockel (18), der mit einem röhrenförmigen Ansatzstück (20) versehen ist, das dazu bestimmt ist, mit einer Öffnung (13) des schalenförmigen Steck-Elements (10) verbunden zu werden,
- ein Halteelement (19) für das Ventil (15), mit dem Luft aus der Schale entweichen kann und das verhindert, dass Luft in das Innere der Schale (10) gelangen kann, welches Halteelement (19) am Sockel (18) angelenkt und beweglich ist zwischen einer angenäherten Position, in der das Halteelement (19) an den Sockel (18) gedrückt wird, und einer entfernten Position, in der das Ventil (15) von der Öffnung (13) entfernt ist, damit das Innere der Schale (10) mit dem Äußeren kommunizieren kann,
- welches Ventil (15) ein elastisches Element mit einer abgeschrägten Aussparung ist, deren Spalt im Allgemeinen geschlossen ist und der sich unter der Wirkung der aus der Schale gepressten Luft öffnet,
**dadurch gekennzeichnet, dass** der Sockel (18) des Verschlusses (14) ferner ausgestattet ist mit:
- einem Einrastelement (25), das mit einer Rückhaltenase (26) des Halteelements (19) in der Weise zusammenwirkt, dass es eine feste Verriegelung des Verschlusses (14) in angenäherter Position sicherstellt, um das Ventil (15) mit der Öffnung (13) zu verbinden,
- und einer Betätigungszunge (29) zum Entriegeln der Rückhaltenase (26) des Halteelements (19) beim Bewegen des Verschlusses (14) in Richtung auf die entfernte Position.

2. Verschluss mit Ventil nach Anspruch 1, **dadurch gekennzeichnet, dass** das Halteelement (19) des Ventils (15) beim Verriegeln elastisch in Richtung auf die entfernte Position gezogen wird.

3. Verschluss mit Ventil nach Anspruch 2, **dadurch gekennzeichnet, dass** im Anlenkbereich des Halteelements (19) und des Sockels (18) eine Feder (22) angeordnet ist.

4. Verschluss mit Ventil nach Anspruch 3, **dadurch gekennzeichnet, dass** die Feder (22) eine Torsionsfeder ist.

5. Verschluss mit Ventil nach einem der vorstehenden Ansprüche, **dadurch gekennzeichnet, dass** das Ventil (15) an den oberen Teil des Halteelements (19) montiert und dass ein Deckel (23) darüber vorgesehen ist, durch den mindestens ein Loch (24) gebohrt ist.

6. Verschluss mit Ventil nach einem der vorstehenden Ansprüche, **dadurch gekennzeichnet, dass** sich das Einrastelement (25) und die Rückhaltenase (26) entgegengesetzt zum Anlenkbereich des Halteelements (19) befinden.

7. Verschluss mit Ventil nach einem der vorstehenden Ansprüche, **dadurch gekennzeichnet, dass** das Halteelement (19) und der Sockel (18) von einem einzigen Teil aus Spritzkunststoff gebildet werden.

8. Schale (10) einer Prothese, die vorgesehen ist, eine Hülse (11) aufzunehmen, die am Stumpf eines amputierten Körperglieds angebracht ist, **dadurch gekennzeichnet, dass** sie mit einem Verschluss (14) mit einem Ventil (15) nach einem der vorstehenden Ansprüche versehen ist.

## Claims

1. A plug (14) with an automatic valve (15) for engagement of a negative pressure prosthesis in the form of a shell (10), said plug (14) comprising:
- a fixing base (18) provided with a tubular end-piece (20) designed to be engaged in a fitting opening (13) in the form of a shell (10),
- a support part (19) of the valve (15) performing removal of air from the shell and preventing air from entering into the shell (10), said support part (19) being articulated on the base (18) and being movable by swivelling between a proximate position where the support part (19) is pressed against the base (18) and a separated position where the valve (15) is away from the opening (13) so as to make the inside of the shell (10) communicate with the outside,
- said valve (15) having a bevelled hollowed flexible part provided with a slot which is normally closed and which opens due to the effect of the air expelled from the shell,
**characterized in that** the base (18) of the plug (14) is further equipped:
- with a clip-fastening part (25) collaborating with a latching nose (26) of the support part (19) so as to perform positive latching of the plug (14) in the proximate position to connect the valve (15) to the opening (13),
- and with an actuating tab (29) to perform unlatching of the latching nose (26) of the support part (19) when movement of the plug (14) to the separated position takes place.

2. The plug with a valve according to claim 1, **characterized in that** the support part (19) of the valve (15) is flexibly biased to the separated position when said unlatching takes place.

3. The plug with a valve according to claim 2, **characterized in that** a spring (22) is housed in the articulation area of the support part (19) and of the base (18).

4. The plug with a valve according to claim 3, **characterized in that** the spring (22) is a torsion spring.

5. The plug with a valve according to one of the foregoing claims, **characterized in that** the valve (15) is fitted on the top part of the support part (19), and is capped by a cover (23) in which at least one hole (24) is drilled.

6. The plug with a valve according to one of the foregoing claims, **characterized in that** the clip-fastening part (25) and the latching nose (26) are located opposite the articulation area of the support part (19).

7. The plug with a valve according to one of the foregoing claims, **characterized in that** the support element (19) and base (18) are formed by a single part made from injected plastic material.

8. A shell (10) of a prosthesis designed to receive a sleeve (11) fitted on the stump of an amputated limb, **characterized in that** it is equipped with a plug (14) with a valve (15) according to one of the foregoing claims.
